# EUROPEAN PATENT APPLICATION

(11) **EP 4 711 761 A1**
(43) Date of publication of application: **18.03.2026**
(21) Application number: 25175565.8
(22) Date of filing: 10.05.2025
(51) Int. Cl.: G01N 30/72, G01N 30/88, G01N 33/493, G01N 33/50, G01N 33/68, C12Q 1/04, C12Q 1/10, A61K 35/741

(54) **DIAGNOSTIC METHOD FOR DETERMINING FUNCTIONAL CHANGES IN THE HUMAN GUT MICROBIOTA**

(30) Priority: 17.09.2024 IT 202400020722
(71) Applicant: Biomi Srl, 73100 Lecce (IT)
(72) Inventor: Pompilio Maria Minelli, Mauro, 73100 Lecce (IT)
(74) Representative: Cornacchia, Pierluigi

(57) **Abstract**

The present invention relates to a diagnostic method for determining functional alterations of the human intestinal microbiota, where said determination is made by analysis of particular and selected metabolites present in urine.

## Description

### 1 Field of technology

The present invention is in the field of diagnostic methods and, in particular, concerns a diagnostic method for determining functional changes in the human intestinal microbiota.

### 2 State of the Art

The microbiota is a complex ecosystem, predominantly made up of bacteria, residing in an anatomical site and in symbiosis with the host organism; it contributes to the well-being and health of the human organism: it contains biologically relevant genes and metabolites that condition the development of the host mucosa and homeostasis. Loss of homeostasis can condition the onset of a wide range of disorders. These include diarrhoea, irritable bowel syndrome (IBS), inflammatory bowel disease (IBD), colorectal cancer and even some liver diseases and allergies, as well as diet-related diseases such as obesity, type 2 diabetes or coeliac disease. The altered composition of the gut microbiota also affects the central nervous system, because the gut and brain are connected by a multitude of communication pathways used by bacterial transmitters and metabolites. It is therefore not surprising that even mental and neurodevelopmental disorders such as depression, anxiety and autism can be linked to dysbiosis of the gut microbiota.

When the intestinal microorganisms are in balance, the condition is called 'eubiosis'. When, on the other hand, the balance is disturbed, a condition called 'intestinal dysbiosis' is established. Intestinal dysbiosis (or dysmicrobism) is an alteration in the quantitative and/or qualitative composition of the intestinal microbiota.

The Russian scientist Elie Metchnikoff (1845 - 1916) popularised the idea of 'dys- symbiosis' or 'dysbiosis', which describes an imbalance in the ecosystem of the gastrointestinal tract. The loss of microbial balance leads to the proliferation of opportunistic, or 'bad' bacteria and mitigates the effects of the predominant 'good' bacteria necessary for a healthy gut.

An unconscious diet (too much sugar and protein, wrong food combination, reduced and bad chewing), the use of laxatives, antacids, antibiotics, multiple therapies create favourable conditions for the development of various toxic substances and putrefaction bacteria, both of which are very harmful.

Intestinal bacteria metabolise food and/or endogenous substrates, resulting in metabolites that are absorbed and excreted with urine, with or without further modification by other organs. The presence of excessive amounts of certain metabolites in the urine may be indicative of bacterial overgrowth

### CURRENT DIAGNOSTIC METHOD

Currently, there is a simple test on a 10 ml sample of urine to assess the presence of altered intestinal bacterial flora. This test consists of quantifying two substances present in the urine by means of colorimetric and high-performance chromatographic methods: scatol and indica, metabolites of tryptophan.

These substances, which are normally present in traces (4 -20 mg) in the urine of subjects with eubiosis, are increased in the case of dysbiosis and give indications as to which part of the intestine suffers most from an imbalance of the bacterial flora: indica is the biomarker of an alteration of the small intestine, while scatol is indicative of an imbalance of the microbiota of the large intestine.

The dosage of these two metabolites makes it possible to check for the presence of fermentative and/or putrefactive phenomena in the intestine: a high level of urinary Indican is mostly indicative of fermentative intestinal dysbiosis in the small intestine, while high levels of Scatol indicate putrefactive intestinal dysbiosis in the large intestine.

### CURRENT TREATMENT STRATEGIES

It is currently possible to assess the presence of a quantitative and qualitative imbalance in the intestinal flora using a direct method of characterising the micro- organisms that physiologically inhabit our intestines and contribute to the absorption and digestion of food consumed. The method involves the use of modern applications of molecular biology, through massive DNA sequencing technologies (High Throughput DNA Sequencing or HTS). One of these methodologies is defined as 'metabarcoding': it indicates the use of a marker gene or barcode (or at least a specific region of this gene) capable of discriminating between the different micro- organisms of the community to be studied.

The analysis represents an important first-level screening to assess the general composition of the resident microbiota, providing adequate prerequisites to identify the best strategies for a correct balance of the bacterial species examined.

Treatment options for dysbiosis include the use of specific probiotics, microorganisms that, when taken in adequate concentrations, exert a positive effect on intestinal well-being and thus on the health of the host organism. These strategies, combined with the contribution of appropriate dietary regimes, are complemented and enriched by the use of prebiotics, i.e. nutrients that provide the substrate for the growth of probiotics and improve the metabolic activity of the gut microbiota, increasing the production of short-chain fatty acids and the proliferation of beneficial species.

The most commonly used probiotics belong to various genera (Lactobacillus, Bifidobacterium, yeasts such as Saccharomyces) and the beneficial effects are manifold in cases of diarrhoea, colitis, inflammatory diseases.

They can be taken as food components, usually dairy products, or as commercial preparations (supplements).

Obviously, the effectiveness of different probiotics in various diseases depends largely on the properties of the bacterial strain used: each clinical condition requires a specific microorganism.

Parallel to the characterisation of bacterial strains, considering that all bacteria or fungi that inhabit the gut produce metabolites that have the ability to modulate cellular activities, depending on the food ingested, it is also important to conduct research on a panel of bacterial metabolites on urinary matrix.

This investigation is not a substitute for microbiota analysis, but will be supplementary, as urinary biomolecules are considered to be true functional markers and their presence will provide additional information, allowing a unique therapeutic proposal to be implemented, even in dietary patterns.

For example, when a sufficient and adequate number of probiotics colonise the gut, the diversity of the gut microbiota can be improved, as the growth of bacterial strains producing short-chain fatty acids (SCFA), which have a prebiotic effect, is promoted, strengthening the intestinal barrier and inhibiting inflammation.

### 3 Summary of the invention

The problem addressed by the present invention is therefore to provide an improved diagnostic method for determining functional changes in the human gut microbiota.

As an additional problem, one would like to have a relatively simple diagnostic method that employs common analytical instrumentation and possibly provides a clear picture of functional alterations in the human gut microbiota with a simple brief analysis

These problems are solved by the method of the present invention as outlined in the appended claims, the definitions of which form an integral part of this description.

In particular, the invention relates to a diagnostic method for determining functional changes in the human intestinal microbiota.

Further features and advantages of the method of the invention will result from the description of the invention, provided as an indication of the invention.

### 4 Detailed description of the invention

As used herein, the term "and/ or", when used in a list of two or more items, means that any one of the listed items may be used alone, or in any combination of two or more of the listed items.

For example, if a composition is described as containing components A, B and/or C, the composition may contain only A; only B; only C; A and B in combination; A and C in combination; B and C in combination; or A, B and C in combination.

The terms "includes", "comprising", or any variation thereof, are intended to cover a non-exclusive inclusion, such that a method, use, or apparatus that includes a list of elements does not include only those elements but may include other elements not expressly listed or inherent in that method, use, or apparatus.

An element followed by "includes... a..." does not prevent, without further constraint, the existence of further identical elements in the method, use or apparatus comprising the element.

An object of the present invention is a diagnostic method for determining functional changes in the human intestinal microbiota. Furthermore, by means of said determination, it is possible to design and field customised preventive and/or therapeutic treatment methods comprising variously modulated combinations of intestinal bacterial species considered probiotic.

An object of the present invention is thus a diagnostic method for determining functional changes in the human intestinal microbiota comprising the analysis of a urine sample directed at determining the quantitative content of the following substances:
(a) tryptophan metabolites such as indica and scatol,
and at least two substances chosen from the following group of substances:
(b) D-lactate,
(c) D- Arabinitol
(d) 3,4-dihydroxy-phenylpropionate,
(e) Lipopolysaccharide (LPS),
(f) trimethylamine oxide (T-MAO).

Among the vast number of metabolites in human urine, it was indeed surprising to find which specific and selected metabolites to analyse and quantify so that functional alterations in the human intestinal microbiota could be determined through them.

In fact, there are a multitude of chemicals in the urine and it is not known which of them need to be monitored and quantified to diagnose functional changes in the human gut microbiota, as the correlation of the urine content of the specific metabolite with the functions in the microbiota is not known.

The diagnostic method of the invention provides for analysing a specific and selected panel of metabolites of the intestinal bacterial flora, In particular, the method provides for analysing the presence and/or quantification in urinary matrix not only of tryptophan metabolites (indica and scatol), but also of
b. D-lactate: represents an important metabolic product of various bacterial strains within the human gut, as human tissues can produce D-lactic acid in extremely small quantities (only in nanomolar concentrations). Fermentative bacterial strains, many of which belong to the genus Lactobacillus Acidophilus, produce D-lactate.
c. D- Arabinitol: is a typical metabolite of Candida Albicans. Its identification in urine provides a clue to the existence of candidiasis and its severity, giving important qualitative and quantitative data.
d. 3,4-dihydroxy-phenylpropionate: derives from phenylalanine metabolism and its presence in high concentrations in urine may reflect an overgrowth of putrefactive anaerobic bacteria, such as Clostridium, which metabolises the aromatic amino acid phenylalanine into phenolic compounds.
e. Lipopolysaccharide (LPS): a pro-inflammatory metabolite that is a component of the outer membrane of gram-negative bacteria.
f. trimethylamine oxide (T-MAO) which is an amine that can promote atherosclerosis and is a product of the metabolism of L-carnitine, found in abundance in red meat.

The diagnostic method of the invention is performed in vitro, i.e. outside the human body.

The diagnostic method of the invention is in fact performed by analysing urine present outside the human body.

Preferably, in the method of the invention, the quantitative content determination is carried out for at least three of substances (b), (c), (d), (e) and (f).

Even more preferably, in the method of the invention, the quantitative content determination is performed for all substances (b), (c), (d), (e) and (f).

According to a preferred aspect, the determination of the quantitative content of substances is carried out either by HPLC or by HPLC/MS or HPLC/MS-MS, where HPLC/MS means High Performance Liquid Chromatography coupled with a mass detector, i.e. a mass spectroscope.

According to a preferred aspect, if the quantitative content of substances is determined by HPLC, the HPLC detector is a UV-Visible detector and/or a refractive index detector.

According to a preferred aspect, where the quantitative content of substances is determined by HPLC, this determination is carried out by means of a single analysis of a urine sample.

### HIGH RESOLUTION CHROMATOGRAPHY - HPLC

High Resolution Chromatography (HPLC, an acronym for High Performance Liquid Chromatography, formerly called High Pressure Liquid Chromatography) is an extremely versatile technique that allows a wide variety of molecules of biological interest to be separated and assayed, even those of high molecular weight. A whole universe of columns for HPLC is available today that allow practically any molecule (or class of molecules) to be analysed by suitably combining the mobile phase (eluent) with the different types of stationary phase (column): normal phase silica, reverse phase silica, ion exchange, gel filtration, gel permeation, chiral phase (for the separation of isomers), etc.

The particles that make up the stationary phase (i.e. the filling of the column) can be of different sizes (particles up to 3 µ m in diameter are available, ensuring extremely efficient separation). The applications in the field of drug dosing are varied.

The chromatographic part of the HPLC instrument can be coupled to a wide variety of detectors (sometimes more than one in sequence), the main ones being the UV-visible spectrophotometer, the diode-array, the fluorometer or spectrofluorometer, the electrochemical detector, etc.

With regard to examinations aimed at MME diagnostics, HPLC is widely used.
Some limited examples of applications are:
- purines and pyrimidines
- homocystein
- ketone bodies
- creatine and guanidinoacetate
- orotic acid and orotidine
- tryptophan and its metabolites
- nucleic acids
- amino acids

Liquid chromatography has recently, following the implementation of the electrospray interface, been successfully coupled to mass spectrometry (particularly Tandem). The use of chromatography columns upstream of the mass spectrometer further enhances the potential of this instrumentation.

### LC/MS-MS

Liquid chromatography techniques interfaced with tandem mass spectrometry provide optimal results and more stability, precision and sensitivity in measuring small concentrations of urinary metabolites in solution in organic acids for.

COLORIMETRIC METHODS: Based on the formation of coloured protein complexes with certain reagents.

Thanks to the metabolomic analyses described above, quantifying the aforementioned specific metabolites in urine will also make it possible to improve the dosage of probiotic therapy and the accuracy of the same or to improve the dosage of probiotic preventive therapy and the accuracy of the same

Another object is thus a bacterial or probiotic composition for use in a method of therapeutic and/or preventive treatment of alterations in the human intestinal microbiota, where this composition is determined qualitatively and/or quantitatively on the basis of the results obtained through the diagnostic method as described above.

The analytical device of HPLC or HPLC/MS has been widely commercially available for several decades now, so the practical realisation of the method of the invention is within the common skills of the analytical chemist and requires no additional inventive effort.

## Claims

1. A diagnostic method for determining functional changes in the human intestinal microbiota comprising the analysis of a urine sample aimed at determining the quantitative content of the following substances:
(a) tryptophan metabolites such as indica and scatol, and of at least two substances chosen from the group of the following substances:
(b) D-lactate,
(c) D- Arabinitol,
(d) 3,4-di-hydroxy-phenylpropionate,
(e) Lipopolysaccharide (LPS),
(f) trimethylamine oxide (T-MAO).

2. A diagnostic method according to claim 1, where said method is performed in vitro.

3. A diagnostic method according to any of the previous claims 1 to 2, wherein the quantitative content determination is performed for at least three of substances (b), (c), (d), (e) and (f).

4. A diagnostic method according to any of the previous claims 1 to 2, wherein the quantitative content determination is performed for all of substances (b), (c), (d), (e) and (f).

5. A diagnostic method according to any of the previous claims 1 to 4, wherein the determination of the quantitative content of the substances is performed by HPLC or by HPLC/MS or HPLC/MS-MS.

6. A diagnostic method according to claim 5, wherein, where the determination of the quantitative content of the substances is carried out by HPLC, the detector of the HPLC is a UV-Visible detector and/or a refractive index detector.

7. A diagnostic method according to any of the previous claims 5 to 6, where, if the determination of the quantitative content of substances is made by HPLC, said determination is made by a single analysis of a urine sample.

8. A bacterial or probiotic composition for use in a method of the therapeutic and/or preventive treatment of alterations in the human intestinal microbiota, wherein said composition is determined qualitatively and/or quantitatively on the basis of the results obtained by the diagnostic method according to any one of the previous claims 1 to 7.
